# EUROPEAN PATENT SPECIFICATION

(11) **EP 2 099 393 B1**
(45) Date of publication and mention of the grant of the patent: **30.06.2010**
(21) Application number: 07845096.2
(22) Date of filing: 15.11.2007
(51) Int. Cl.: A61F 2/90

(54) **BIFURCATED STENT WITH VARIABLE LENGTH BRANCHES**
GEGABELTER STENT MIT ÄSTEN VARIABLER LÄNGE
ENDOPROTHÈSE VASCULAIRE AVEC BIFURACTION AYANT DES BRANCHES DE LONGUEUR VARIABLE

(30) Priority: 01.12.2006 US 565947
(43) Date of publication of application: 16.09.2009
(73) Proprietor: Medtronic Vascular, Inc., Santa Rosa, CA 95403 (US)
(72) Inventor: MOLONEY, Noreen, Galway (IE)
(74) Representative: Hughes, Andrea Michelle
(86) International application number: PCT/US2007/084842
(87) International publication number: WO 2008/070423

(56) References cited:
- WO-A-01/43810
- US-A- 5 713 917
- US-A1- 2003 191 518
- US-B1- 7 112 217

## Description

### FIELD OF THE INVENTION

The present invention relates generally to bifurcated stents. More particularly, the present invention is directed to a bifurcated stent wherein the length of the branches of the stent can be adjusted prior to implantation.

### BACKGROUND OF THE INVENTION

A number of medical procedures involve or can be supplemented with the placement of an endoluminal prosthesis, commonly referred to as a stent, that can be implanted in a lumen, such as a blood vessel or other natural pathway of a patient's body. Such stents typically define a generally tubular configuration, and are expandable from a relatively small diameter (low profile) to an enlarged diameter. While in its low profile configuration, the stent is advanced endoluminally, by a delivery device, through the body lumen to the site where the stent is to be placed. The stent then can be expanded to a larger diameter in which it can firmly engage the inner wall of the body lumen. The delivery device then is removed, leaving the implanted stent in place. In that manner, the stent may serve to maintain open a blood vessel or other natural duct, the functioning of which had become impaired as a result of a pathological or traumatic occurrence.

Among the medical procedures in which stents have had increasing use is in connection with percutaneous transluminal angioplasty (PTA), and particularly percutaneous transluminal coronary angioplasty (PTCA). PTA and PTCA involve the insertion and manipulation of a dilating catheter through the patient's arteries to place the dilatation balloon of the catheter within an obstructed portion (stenosis) of a blood vessel. The balloon then is expanded forcibly within the obstruction to dilate that portion of the blood vessel thereby to restore blood flow through the blood vessel. Among the more significant complications that may result from such angioplasty is that in a significant number of cases, the dilated site again becomes obstructed. By placing a stent within the blood vessel at the treated site, the tendency for such restenosis may be reduced.

Stenoses often may develop in the branching region of a patient's blood vessel. Treatment of a stenosis in the branched region may present numerous additional difficulties in the design of devices to dilate stenoses at the branched region. Techniques and devices have been developed to effect a dilatation at a branched region such as the "kissing balloon" technique described in U.S. Pat. No. 4,896,670.

A number of stents have been proposed and developed in the art, including single stents that define a single luminal pathway as well as bifurcated stents that define a branched pathway and are intended to be placed in a branching region of a blood vessel. The development of bifurcated stents, as compared to single stents presents numerous difficulties because of the branched arrangement and the difficulty in delivering and placing a bifurcated stent at the branched region of a blood vessel.

U.S. Pat. No. 4,994,071 (MacGregor) discloses a design for a bifurcating stent intended to be inserted into a bifurcated blood vessel. The stent is constructed from two lengths of continuous wire, one of which is formed in a series of interconnected loops to define a common tubular branch and one of the bifurcated branches. The other length of wire also is formed in a series of similarly interconnected loops to define the other branch of the bifurcation. The two assemblies of interconnected loops are connected together to define a Y-shaped structure. The interconnection between the structure defining the bifurcated branches is said to enable them to be bent to conform to the shape of the vessels into which the device is intended to be inserted. The loops are formed so that they can be expanded from an initial diameter to facilitate insertion into the blood vessel to an expanded, deployed diameter. US 7112217 teaches a bifurcated stent-graft system including a telescopic mechanism to permit in-situ length adjustment.

The MacGregor and other bifurcated devices present a number of difficulties. Its continuous wire construction does not readily lend itself to precise matching to the vascular anatomy of pathological situation of the specific patient in whom the stent is to be placed. The construction is adapted, as a practical matter, only to be manufactured in standard configurations and lengths. When a standard length of stent does not ideally match the patient's anatomy, the physician would be forced to choose among the available standard lengths and configurations in an effort to make a selection that, at best, could be considered to be a compromise.

### BRIEF SUMMARY OF THE INVENTION

A bifurcated stent is disclosed including a trunk portion and first and second branches. At least one of the branches includes a longitudinally extendable portion such that the branch can be extended from a first length up to second length. In one embodiment, the trunk portion includes a plurality of cylindrical rings coupled to each other, and each of the first and second branches includes a plurality of cylindrical rings coupled to each other. The cylindrical rings of the longitudinally extendable portion are coupled to each other by a link with a curved portion. Upon pulling of the branch, the link with the curved portion straightens, thereby lengthening the branch.

In another embodiment, the trunk portion is formed from a continuous wire formed into a zig-zag pattern and spirally wound around a mandrel to form a cylindrical body. The first branch is also formed from a continuous wire formed into a zig-zag pattern and spirally wound around a mandrel to form a cylindrical body. The second branch is also formed from a continuous wire formed into a zig-zag pattern and spirally wound around a mandrel to form a cylindrical body. At least one of the first and second branches includes a longitudinally extendable portion. The longitudinally extendable portion is formed by winding the continuous wire of the branch at a first pitch whereas the remainder of the branch is wound at a second pitch greater than the first pitch. Thus, when the branch with the longitudinally extendable portion is pulled, the pitch of the longitudinally extendable portion increases, thereby lengthening the branch.

A method of treating a stenosis at a branched vessel of a patient is also disclosed. The method includes determining the location and the size of the stenosis. Stents are normally provided already mounted on the delivery system. In an embodiment, the delivery system is a balloon catheter. After determining the size of the stenosis, a stent and delivery system combination is selected. Based on the size of the stenosis, one or both of the branches may be longitudinally extended to fit the length of the stenosis in the particular branch of the vessel. The stent and delivery system are inserted into the vessel and advanced to the site of the stenosis. The balloon(s) of the balloon catheter is inflated to expand at least a portion of the stent. However, because at least a portion of the stent was lengthened, the balloon may not expand the entire stent. Therefore, the balloon is deflated, relocated to the unexpanded portion of the stent, and re-inflated to expand the unexpanded portion of the stent. The balloon(s) is then deflated and the delivery system is withdrawn from the vessel. In an alternative embodiment, the balloon(s) of the balloon catheter is also provided with a longitudinally extendable portion such that when the stent is lengthened, the balloon(s) is also lengthened. Such an embodiment eliminates the steps of deflating, relocating, and re-inflating the balloon(s).

### BRIEF DESCRIPTION OF DRAWINGS

The foregoing and other features and advantages of the invention will be apparent from the following description of the invention as illustrated in the accompanying drawings. The accompanying drawings, which are incorporated herein and form a part of the specification, further serve to explain the principles of the invention and to enable a person skilled in the pertinent art to make and use the invention. The drawings are not to scale.

FIG. 1 illustrates a plan view of a bifurcated stent which has been cut and laid open.

FIG. 2 illustrates a plan view of the stent of FIG. 1 with one of the branches extended.

FIG. 3 illustrates a plan view of another embodiment of a bifurcated stent which has been cut and laid open.

FIG. 4 illustrates a plan view of the stent of FIG. 3 with one of the branches extended.

FIG. 5 illustrates a plan view of a portion of a stent.

FIG. 6 illustrates a plane view of a portion of a stent.

FIG. 7 illustrates a perspective view of a stent of another embodiment of the present invention.

FIG. 8 illustrates a perspective view of the stent of FIG. 7 with one of the branches extended.

FIG. 9 is a diagram illustrating a method of making delivering an extendeble stent of the present invention to a treatment site.

FIG. 10 illustrates a cross-sectional view of a delivery system for an extendable stent of the present invention.

FIG. 11 illustrates a cross-sectional view of the delivery system of FIG. 10 with a stent superimposed on the delivery system.

### DETAILED DESCRIPTION OF THE INVENTION

Specific embodiments of the present invention are now described with reference to the figures, where like reference numbers indicate identical or functionally similar elements. While specific embodiments are discussed in detail, it should be understood that this is done for illustrative purposes only. A person skilled in the art will recognize that other embodiments can be used without departing from the scope of the invention.

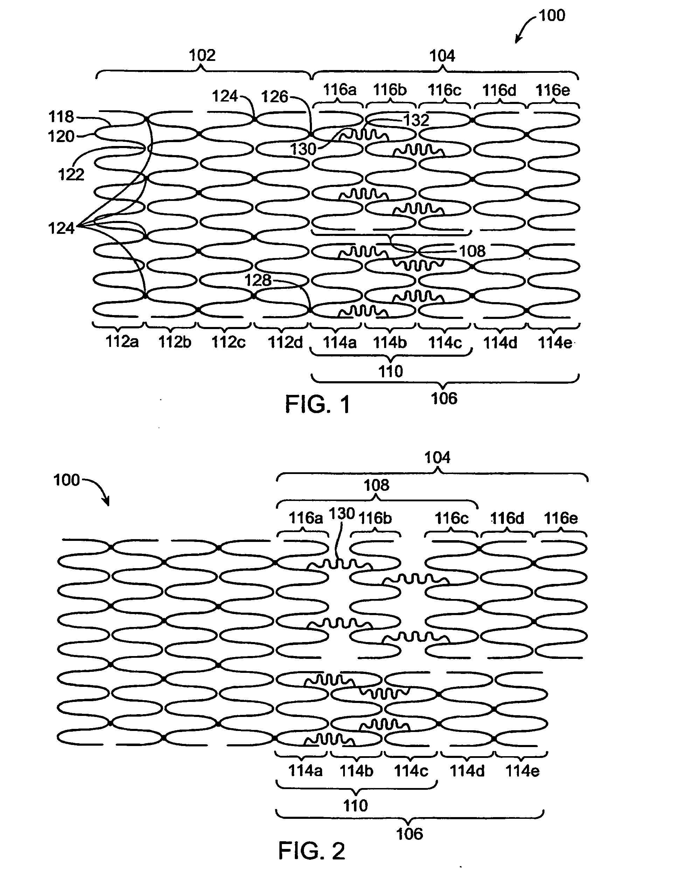

FIGS. 1 and 2 illustrate a plan view of a bifurcated stent 100, which has been cut and laid open for illustrative purposes. In its unaltered state, stent 100 is generally hollow and cylindrical in shape, as known to those of ordinary skill in the art. Stent 100 includes a trunk 102, a first branch 104, and a second branch 106. Stent 100 is a bifurcated stent such that trunk 102 is designed to fit into a main vessel and first and second branches 104 and 106 are designed to fit into branch vessels extending from the main vessel, sometimes referred to as a main branch and a side branch. Trunk 102 includes rings 112*a*-112*d*. Each of rings 112*a*-112*d* extends around the cylindrical body of stent 100 and includes an undulating series of peaks 122 and valleys 120 connected together by segments 118. Although four (4) rings 112*a*-112*d* are shown in FIGS. 1 and 2, it would be understood one of ordinary skill in the art that any desirable amount of rings may be used, depending on the nature and location of the stenosis. Similarly, first branch 102 comprises rings 116*a-*116e and second branch 104 comprises rings 114*a*-114*e*, each including an undulating series of peaks 122 and valleys 120 connected together by segments 118.

Rings 112*a*-112*d* are coupled together at weld points 124 connecting a peak of one ring to a valley of an adjacent ring. In the embodiment shown in FIGS. 1 and 2, every other peak of one ring is connected to the corresponding valley of the adjacent ring. For example, ring 112*a* is coupled to ring 112*b* at four (4) weld points 124. While weld points 124 are shown in FIGS. 1 and 2 to connect rings 112*a*-112*d* together, one of ordinary skill in the art would understand that other ways of coupling the rings may be used, for example, links may be used. Links may connect peak to valley, peak to peak, or may connect from a segment to an adjacent segment or to a peak or valley. Similarly, while the embodiment of FIGS. 1 and 2 shows that the undulations of rings 112*a*-112*d* are "out of phase" by 180 degrees with adjacent rings, such that a peak of one ring matches up with a valley of an adjacent ring, the rings may be "in phase" such that peaks align with peaks and valleys align with valleys, or the rings may be out of phase by an amount less than 180 degrees.

First branch 104 and second branch' 106 are coupled to trunk 102 at weld points 126 and 128, respectively. As would be understood by one of ordinary skill in the art, other ways of connecting branches 104 and 106 to trunk 102 may be used, such as links. Further, while one (1) weld point is shown for each branch to trunk connection, it would be understood that more than one connection can be used.

First branch 104 includes a longitudinally extendable portion 108. In the embodiment of FIGS. 1 and 2, longitudinally extendable portion 108 includes rings 116*a*, 116*b*, and 116*c*. Ring 116*a* is coupled to ring 116*b* and ring 116*b* is coupled to ring 116*c* by links 130. In the embodiment shown in FIGS. 1 and 2, links 130 include curved portions 132. Similarly, second branch 106 includes a longitudinally extendable portion 110. In the embodiment of FIGS. 1 and 2, longitudinally extendable portion 110 includes rings 114*a*, 114*b*, and 114*c*. Ring 114*a* is coupled to ring 114*b* and ring 114*b* is coupled to ring 114*c* by links 130. As would be understood by one of ordinary skill in the art, extendable portions 108 and 110 may include more or less rings, although at least two rings would generally be necessary to form an extendable portion. In the embodiment shown in FIGS. 1 and 2, rings 116*c*-116*e* and rings 114*c*-114*e* are coupled to each other at weld points 124.

In practice, stent 100 can be used at branched vessels with stenoses of different lengths without having to use a different stent. A physician can view the stenosis and adjust the length of one or both branches 104, 106 by pulling on the branch. As seen in FIG. 2, first branch 104 has been pulled, thereby resulting in links 130 straightening and rings 116*a*-116*c* of longitudinally extendable portion 108 separating from each other. Thus, first branch 104 has effectively become longer and covers a longer stenosis. Because rings 116*a*-116*c* have been separated, coverage of the stenosis in the areas between the rings is reduced. However, in the embodiment shown, only two links 130 have been provided between rings 116*a* and 116*b* and two links 130 between rings 116*b* and 116*c*. One of ordinary skill in the art would understand that in order to increase the coverage, more links 130 can be provided between the rings.

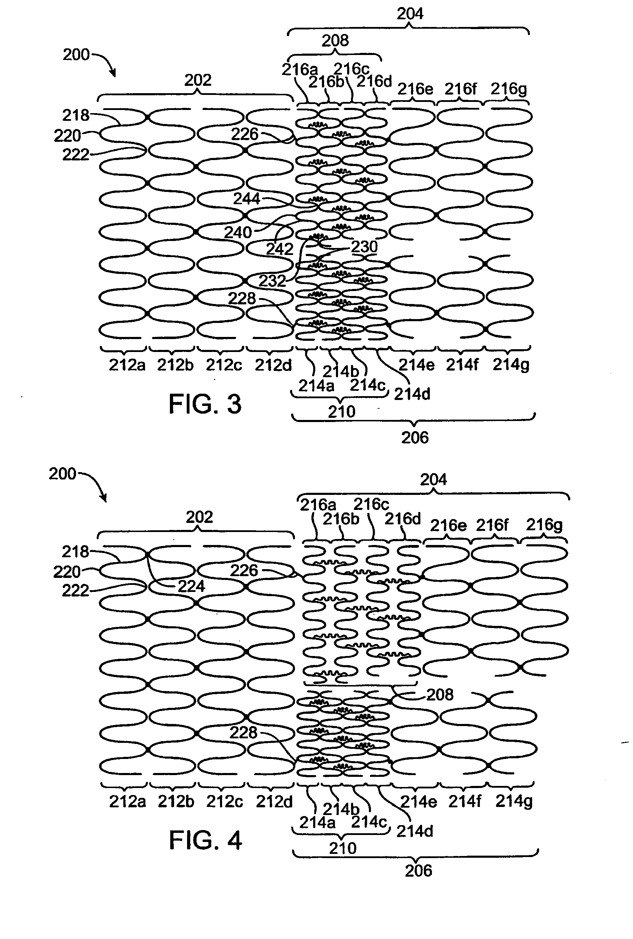

Another way to increase coverage in the longitudinally extendable portions of the branches of a bifurcated stent is shown in another embodiment of the present invention shown in FIGS. 3 and 4. FIGS. 3 and 4 illustrate a plan view of a bifurcated stent 200, which has been cut and laid open for illustrative purposes. Similar to stent 100 of FIGS. 1 and 2, stent 200 includes a trunk 202, a first branch 204, and a second branch 206. Stent 200 is a bifurcated stent such that trunk 202 is designed to fit into a main vessel and first and second branches 204 and 206 are designed to fit into branch vessels extending from the main vessel, sometimes referred to as a main branch and a side branch. Trunk 202 includes rings 212*a-*212*d*. Each of rings 212*a*-212*d* extends around the cylindrical body of stent 200 and includes an undulating series of peaks 222 and valleys 220 connected together by segments 218. Although four (4) rings 212*a*-212*d* are shown in FIGS. 3 and 4, it would be understood one of ordinary skill in the art that any desirable amount of rings may be used, depending on the nature and location of the stenosis. Similarly, first branch 202 comprises rings 216*a*-216*g* and second branch 204 comprises rings 214*a*-214*g*, each including an undulating series of peaks and valleys connected together by segments.

Rings 212*a*-212*d* are coupled together at weld points 224 connecting a peak of one ring to a valley of an adjacent ring. In the embodiment shown in FIGS. 3 and 4, every other peak of one ring is connected to the corresponding valley of the adjacent ring. For example, ring 212*a* is coupled to ring 212*b* at four (4) weld points 224. While weld points 224 are shown in FIGS. 3 and 4 connecting rings 212*a*-212*d* together, one of ordinary skill in the art would understand that other ways of coupling the rings may be used, for example, links may be used. Links may connect peak to valley, peak to peak, or may connected from a segment to an adjacent segment or to a peak or valley. Similarly, while the embodiment of FIGS. 3 and 4 shows that the undulations of rings 212*a*-212*d* are "out of phase" by 180 degrees with adjacent rings, such that a peak of one ring matches up a valley of an adjacent ring, the rings may be "in phase" such that peaks align with peaks and valleys align with valleys, or the rings may be out of phase by an amount less than 180 degrees.

First branch 204 and second branch 206 are coupled to trunk 202 by connecting links 226 and 228, respectively. As would be understood by one of ordinary skill in the art, other ways of connecting branches 204 and 206 to trunk 202 may be used, such as welds shown in FIGS 1 and 2. Further, while one (1) link is shown for each branch to trunk connection, it would be understood that more than one link can be used for each connection.

First branch 204 includes a longitudinally extendable portion 208. In the embodiment of FIGS. 3 and 4, longitudinally extendable portion 108 includes rings 216*a*, 216*b*, 216*c*, and 216*d*. Ring 216*a* is coupled to ring 216*b*, ring 216*b* is coupled to ring 216*c*, and ring 216*c* is coupled to ring 216*d* by links 230. In the embodiment shown in FIGS. 3 and 4 links 230 include curved portions 232. Similarly, second branch 206 includes a longitudinally extendable portion 210. Longitudinally extendable portion 210 includes rings 214*a*, 214*b*, 214*c*, and 214*d*. Ring 214*a* is coupled to ring 214*b*, ring 214*b* is coupled to ring 214*c*, and ring 214*c* is coupled to ring 214*d* by links 230. Extendable portions 208 and 210 are similar to extendable portions 108 and 110 of FIGS. 1 and 2, except that rings 216*a*-216*d* and 214*a*-214*d* of extendable portions 208 and 210 are denser than the remaining rings 216*e*-216*g* and 214*e*-214*g* of first and second branches 204 and 206. In other words, as shown in FIGS. 3 and 4 rings 216*a*-216*d* and 214*a*-214*d* include peaks 244, valleys 240, and segments 242 connecting the peaks 244 and valleys 240, similar to peaks 222, valleys 220, and segments 218. However, segments 242 are shorter longitudinally than segments 218. Further, the radius of the bends of peaks 244 and valleys 240 is smaller than the radius of the bends of peaks 222 and valleys 220. Accordingly, a quantity of rings 216*a*-216*d* and 214*a*-214*d* is shorter longitudinally than the same quantity of rings 216*e*-216*g* and 214*e*-214*g*. Further, more peaks 244 and valleys 240 are required to create a cylindrical body of the same diameter as a cylindrical body created by peaks 222 and valleys 220. For example, in the embodiment of FIGS. 3 and 4, each ring 216*e*-216*g* includes a total of nine (9) peaks and valleys, whereas each rig 216*a*-216*d* includes 16 peaks and valleys. As would be understood by one of ordinary skill in the art, extendable portions 208 and 210 may include more or less rings, although at least two rings would generally be necessary to form an extendable portion. In the embodiment shown in FIGS. 3 and 4, rings 216*e*-216*g* and rings 214*e*-214*g* are coupled to each other at weld points 224.

Similar to FIGS. 1 and 2, stent 200 of the embodiment of FIGS. 3 and 4 can be used at branched vessels with stenoses of different lengths without having to use a different stent. A physician can view the stenosis and adjust the length of one or both branches 204, 206 by pulling on the branch. As seen in FIG. 4, first branch 204 has been pulled, thereby resulting in links 230 straightening and rings 116*a-*116*d* of longitudinally extendable portion 108 separating from each other. Thus, first branch 204 has effectively become longer and covers a longer stenosis. Because rings 216*a*-216*d* are denser than rings 216*e*-216*g* coverage of the stenosis in the area of extendable portion 208 is not compromised despite rings 216a-216d being pulled away from each other.

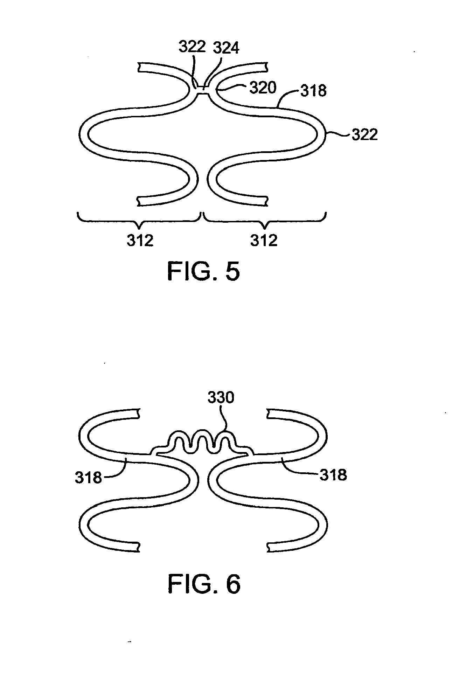

In the embodiments shown in FIGS. 1-4, the rings are welded to each other. However, one of ordinary skill would recognize that there are several ways to make the stents described in FIGS. 1-4. For example, in a typical method of making a stent, a thin-walled, small diameter metallic tube is cut to produce the desired stent pattern, using methods such as laser cutting or chemical etching. The cut stent may then be descaled, polished, cleaned and rinsed. The trunk and each branch of the stents described could be made using this method, and then coupled by a weld or link, as shown. Using, such a method, instead of a weld between rings, a link is normally cut out of the tube, as shown in FIG. 5. In FIG. 5, a peak 322, valley 320, and segment 318 connecting the peak and valley of portions of adjacent rings 312 are shown. A link 324 couples a peak 322 of one ring 312 to a valley 320 of an adjacent ring 312. FIG. 6 shows a curved connecting link 330 used to connect a segment 318 of one ring to a segment 318 of an adjacent ring. Such a link can be used for longitudinally extendable portions 108, 110, 208, 210 described in FIGS. 1-4 or for connecting any other adjacent rings in stents 100, 200.

Some examples of other methods of forming stents and structures for stents are shown in U.S. Patent No. 4,733,665 to Palmaz, U.S. Patent No. 4,800,882 to Gianturco, U.S. Patent No. 4,886,062 to Wiktor, U.S. Patent No. 5,133,732 to Wiktor, U.S. Patent No. 5,292,331 to Boneau, U.S. Patent No. 5,421,955 to Lau, U.S. Patent No. 5,935,162 to Dang, U.S. Patent No. 6,090,127 to Globerman, and U.S. Patent No. 6,730,116 to Wolinsky et al.

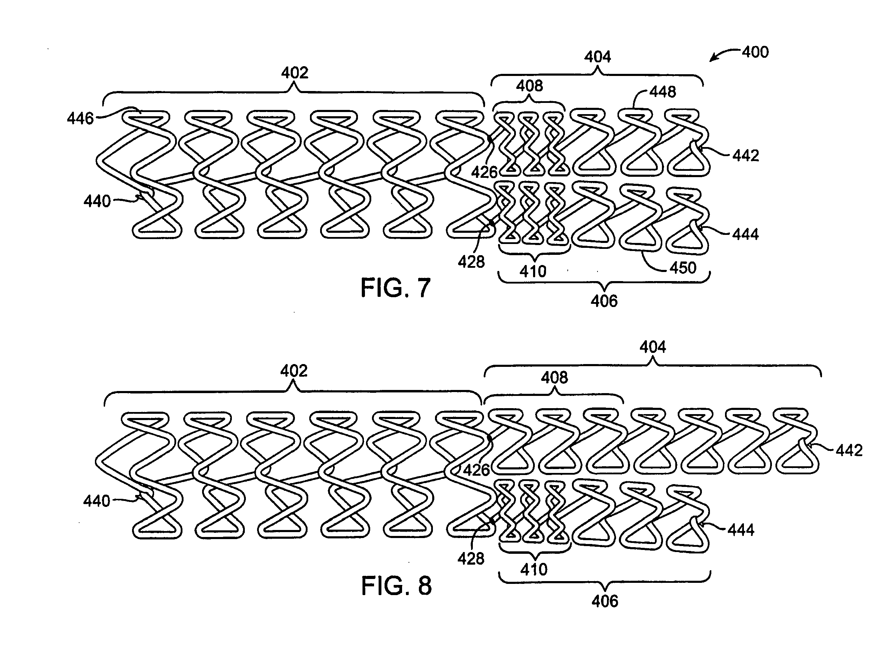

FIGS. 7 and 8 show an embodiment of a spirally wound wire bifurcated stent 400. Stent 400 includes a trunk 402, a first branch 404, and a second branch 406. First branch 404 is coupled to trunk 402 at weld 426 and second branch 406 is coupled to trunk 402 at weld 428. Trunk 402 is formed by a wire 446 formed in a zig-zag shape and then wrapped around a mandrel, as described, for example, in U.S. Patent No. 5,133,732 to Wiktor. A free end 440 of wire 446 may be wrapped around a portion of wire 446 so that free end 440 expands with the remainder of trunk 402. In the alternative, free end 440 may be welded to wire 446, as would be understood by one of ordinary skill in the art. First and second branches 404 and 406 are similarly formed by wires 448 and 450, respectively, formed in a zig-zag shaped and wrapped around a mandrel. Free ends 442 and 444 of wires 448 and 450 are wrapped around a portion of wires 448 and 450, respectively. As with free end 440, free ends 442 and 44 may alternatively be welded.

First branch 404 of stent 400 includes a longitudinally extendable portion 408. Longitudinally extendable portion 408 is formed by wrapping wire 448 around a mandrel at a tighter pitch than the remainder of first branch 404, as can be seen in FIG. 2. Similarly, second branch 406 may include a longitudinally extendable portion 410 formed by wrapping wire 450 around a mandrel at a tighter pitch than the remainder of second branch 406. In practice, if a portion of stenosis in a branch of a branched vessel is longer than the length of first or second branch 404, 406, a physician may pull one of the branches to extend the length thereof. For example, as shown in FIG. 8, first branch 404 may be pulled such that extendable section 408 extends from the tighter pitch of FIG. 7 to the wider pitch of FIG. 8, thereby extending the effective length of first branch 404. As would be understood by one of ordinary skill in the art, second branch 406 may also be extended, or only second branch 406 may be extended and first branch 404 may remain at the length shown in FIG. 7. The term "pitch" as used herein is used as the term is used in relation to coil springs, that is, the distance from center to center of the wire in adjacent coils. Thus, as used herein, a "tighter pitch" has a smaller distance from center to center of the wire in adjacent coils and a "wider pitch" has a larger distance from center to center of the wire in adjacent coils.

The material for the stent of any of the above embodiments may be any material that is typically used for a stent, for example, stainless steel, "MP35N," "MP20N," nickel titanium alloys such as Nitinol, tantalum, platinum-iridium alloy, gold, magnesium, L605, or combinations thereof. "MP35N" and "MP20N" are trade names for alloys of cobalt, nickel, chromium and molybdenum available from standard Press Steel Co., Jenkintown, Pa. "MP35N" consists of 35% cobalt, 35% nickel, 20% chromium, and 10% molybdenum. "MP20N" consists of 50% cobalt, 20% nickel, 20% chromium, and 10% molybdenum.

The stents of the embodiments described may be coated, for example, with a polymer coating. Examples of bioabsorbable, biodegradable materials include but are not limited to polycaprolactone (PCL), poly-D, L-lactic acid (DL-PLA), poly-L-lactic acid (L-PLA), poly(lactide-co-glycolide), poly(hydroxybutyrate), poly(hydroxybutyrate-co-valerate), polydioxanone, polyorthoester, polyanhydride, poly(glycolic acid), poly(glycolic acid-cotrimethylene carbonate), polyphosphoester, polyphosphoester urethane, poly (amino acids), cyanoacrylates, poly(trimethylene carbonate), poly(iminocarbonate), copoly(ether-esters), polyalkylene oxalates, polyphosphazenes, polyiminocarbonates, and aliphatic polycarbonates. Biomolecules such as heparin, fibrin, fibrinogen, cellulose, starch, and collagen are typically also suitable. Examples of biostable polymers include Parylene®, Parylast®, polyurethane (for example, segmented polyurethanes such as Biospan®), polyethylene, polyethlyene terephthalate, ethylene vinyl acetate, silicone and polyethylene oxide.

The stents described above may include a therapeutic substance either directly applied to the stent, in reservoirs in the stent, or as part of a polymer coating, or other ways that would be recognized by one of ordinary skill in the art. Therapeutic substances can include, but are not limited to, antineoplastic, antimitotic, antiinflammatory, antiplatelet, anticoagulant, anti fibrin, antithrombin, antiproliferative, antibiotic, antioxidant, and antiallergic substances as well as combinations thereof. Examples of such antineoplastics and/or antimitotics include paclitaxel (e.g., TAXOL® by Bristol-Myers Squibb Co., Stamford, Conn.), docetaxel (e.g., Taxotere® from Aventis S. A., Frankfurt, Germany), methotrexate, azathioprine, vincristine, vinblastine, fluorouracil, doxorubicin hydrochloride (e.g., Adriamycin® from Pharmacia & Upjohn, Peapack N.J.), and mitomycin (e.g., Mutamycin® from Bristol-Myers Squibb Co., Stamford, Conn.). Examples of such antiplatelets, anticoagulants, antifibrin, and antithrombins include sodium heparin, low molecular weight heparins, heparinoids, hirudin, argatroban, forskolin, vapiprost, prostacyclin and prostacyclin analogues, dextran, D-phe-pro-arg-chloromethylketone (synthetic antithrombin), dipyridamole, glycoprotein IIb/IIIa platelet membrane receptor antagonist antibody, recombinant hirudin, and thrombin inhibitors such as Angiomax™ (Biogen, Inc., Cambridge, Mass.). Examples of such cytostatic or antiproliferative agents include angiopeptin, angiotensin converting enzyme inhibitors such as captopril (e.g., Capoten® and Capozide® from Bristol-Myers Squibb Co., Stamford, Conn.), cilazapril or lisinopril (e.g., Prinivil® and Prinzide® from Merck & Co., Inc., Whitehouse Station, N.J.), calcium channel blockers (such as nifedipine), colchicine, fibroblast growth factor (FGF) antagonists, fish oil (omega 3-fatty acid), histamine antagonists, lovastatin (an inhibitor of HMG-CoA reductase, a cholesterol lowering drug, brand name Mevacor® from Merck & Co., Inc., Whitehouse Station, N.J.), monoclonal antibodies (such as those specific for Platelet-Derived Growth Factor (PDGF) receptors), nitroprusside, phosphodiesterase inhibitors, prostaglandin inhibitors, suramin, serotonin blockers, steroids, thioprotease inhibitors, triazolopyrimidine (a PDGF antagonist), and nitric oxide. An example of an antiallergic agent is permirolast potassium. Other therapeutic substances or agents that may be used include alpha-interferon, genetically engineered epithelial cells, and dexamethasone. In other examples, the therapeutic substance is a radioactive isotope for implantable device usage in radiotherapeutic procedures. Examples of radioactive isotopes include, but are not limited to, phosphorus (P³²), palladium (Pd¹⁰³), cesium (Cs¹³¹), Iridium (I¹⁹²) and iodine (I¹²⁵). While the preventative and treatment properties of the foregoing therapeutic substances or agents are well-known to those of ordinary skill in the art, the substances or agents are provided by way of example and are not meant to be limiting. Other therapeutic substances are equally applicable for use with the disclosed methods and compositions.

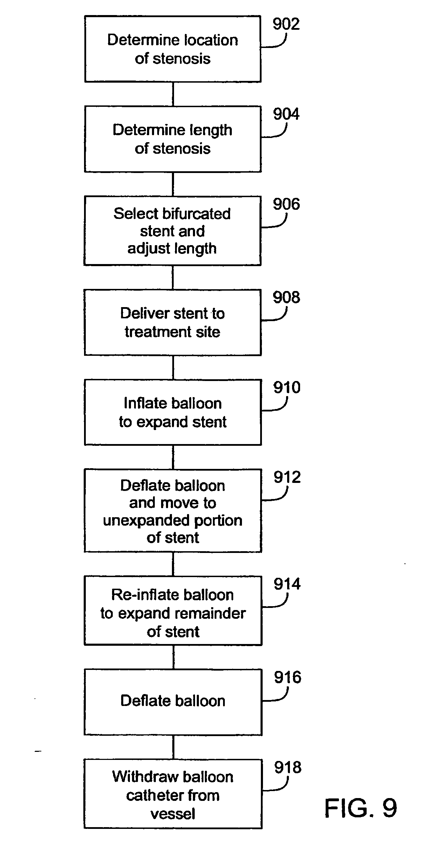

The present invention also relates to a method of delivering a bifurcated stent to a stenosed region of a branched vessel. As shown in FIG. 9, step 902 of the method is a step of determining the location of the stenosis. Step 902 is generally performed angiographically. Step 904 includes assessing the length of the stenosis in the main vessel and the branch vessels, which is also generally done angiographically. Once the location and size of the stenosis has been determined, step 906 includes selecting a bifurcated stent according to the present invention and adjusting the length of one or both of the branches by pulling the stent proximally on the delivery catheter. Step 908 includes delivering the stent and catheter to the site of the stenosis. Step 910 includes inflating the balloon of the balloon catheter to expand the bifurcated stent. Because the stent was extended, the balloon does not expand the entire stent. Therefore, step 912 includes deflating the balloon and moving it to the unexpanded portion of the stent. Step 914 includes re-inflating the balloon to expand the remainder of the stent. Step 916 includes deflating the balloon and step 918 includes withdrawing the balloon catheter from the vessel.

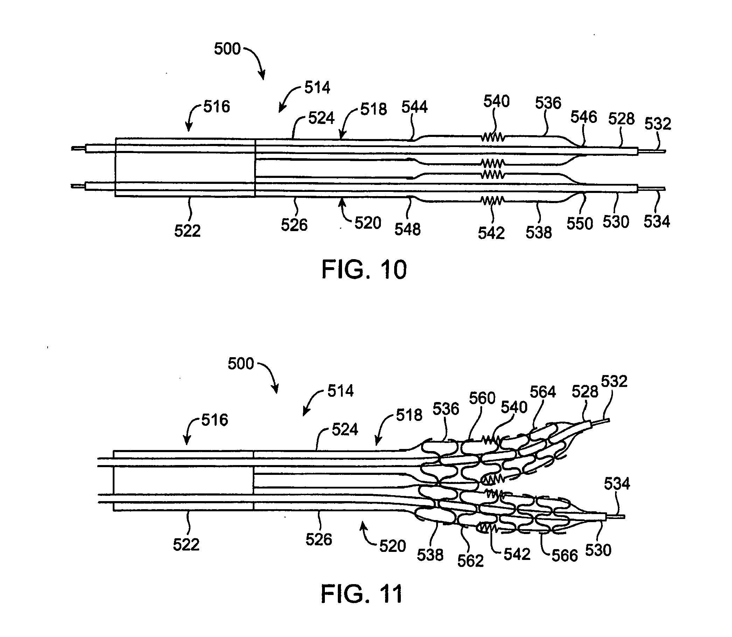

In another embodiment for delivering a bifurcated stent 500 of the present invention, shown in FIGS. 10 and 11, a delivery catheter 510 includes a balloon 512 mounted on a catheter 514. Catheter 514 is a Y-shaped catheter for delivering a bifurcated stent. Accordingly, catheter 514 includes a trunk portion 516, a first branch 518, and a second branch 520. Catheter 514 includes a proximal outer shaft 522, a first branch outer shaft 524, and a second branch outer shaft 526. Catheter 514 further includes a first inner shaft 528 extending at least partially through proximal outer shaft 522 and through first branch outer shaft 524. Catheter 514 also includes a second inner shaft 530 extending at least partially through proximal outer shaft 522 and through second branch outer shaft 526. As would be understood by one of ordinary skill in that art, catheter 514 could also be a rapid exchange catheter, without departing from the scope of the present invention.

As shown in FIGS. 10 and 11, a first balloon 536 is mounted on first branch 518 and a second balloon 538 is mounted on second branch 520. In particular, a proximal neck 544 of first balloon 536 is bonded to a distal portion of first branch outer shaft 524 and a distal neck 546 of first balloon 536 is bonded to a distal portion of first inner shaft 528. Similarly, a proximal neck 548 of second balloon 536 is bonded to a distal portion of second branch outer shaft 526 and a distal neck 550 of second balloon 538 is bonded to a distal portion of second inner shaft 530. First balloon 536 includes a longitudinally extendable portion 540 and second balloon 538 includes a longitudinally extendable portion 542. Longitudinally extendable portions 540 and 542 permit first and second balloons 536 and 538 to extend longitudinally with the longitudinally extendable portions of the stents of the present invention, as described above. In the embodiment of FIGS. 10 and 11, first and second balloons 536 and 538 include bellows type folds to created longitudinally extendable portions 540 and 542, respectively. First inner shaft 528 and second inner shaft 530 extend in conjunction with first and second balloons 536 and 538, respectively.

FIG. 11 shows an exemplary bifurcated stent 560 mounted on the delivery system 500 of the present invention. As shown in FIG. 11, a trunk portion 562 of stent 560 is mounted over first and second balloons 536 and 538. A first branch 564 of stent 560 is mounted over only first balloon 536 and a second branch 566 of stent 560 is mounted on second balloon 538. Such an arrangement is similar to that described in U.S. Patent No. 6,129,738 to Lashinsky et al.

In practice, the location and size of the stenosis are determined, for example, by angiograph. With stent 560 mounted on delivery system 500, stent 560, first and/or second balloons 536 and 538, and first and/or second inner shafts 528 and 530 are extended an appropriate amount to cover the stenosis. Stent 560 is then delivered to the treatment site mounted on delivery system 500. When stent 560 has reached the delivery site, first and second balloons 536 and 538 are inflated to expand stent 560. After stent 560 is in place in its expanded state, first and second balloons 536 and 538 are deflated and delivery system 500 is removed from the vessel, leaving stent 560 in place.

While various embodiments of the present invention have been described above, it should be understood that they have been presented by way of illustration and example only, and not limitation. It will be apparent to persons skilled in the relevant art that various changes in form and detail can be made therein without departing from the scope of the invention. Thus, the breadth and scope of the present invention should not be limited by any of the above-described exemplary embodiments, but should be defined only in accordance with the appended claims. It will also be understood that each feature of each embodiment discussed herein, and of each reference cited herein, can be used in combination with the features of any other embodiment.

## Claims

1. A bifurcated stent comprising:
a trunk portion (102) including a plurality of cylindrical rings (112a-112d) coupled to each other;
a first branch (104) coupled to the trunk portion, wherein the first branch includes a plurality of cylindrical rings (116a-116e) coupled to each other; and
a second branch (106) coupled to the trunk portion, wherein the second branch includes a plurality of cylindrical rings (114a-114e) coupled to each other; **characterized in that**
the first branch includes a longitudinally extendable portion (108),
wherein the longitudinally extendable portion is structured such that a user may increase a length of the first branch prior to installation in a patient.

2. The bifurcated stent of claim 1, wherein the longitudinally extendable portion includes at least two cylindrical rings (11fia-116c) and at least one longitudinal link (130) coupling the at least two cylindrical rings together, wherein the at least one longitudinal link includes a curved portion (132) structured such that pulling the first branch causes the longitudinal link to straighten and a distance between the two cylindrical rings to increase, thereby increasing the length of the first branch.

3. The bifurcated stent of claim 1, wherein each of the cylindrical rings of the trunk portion comprises a series of peaks (122) and valleys (120) coupled together by segments (118), wherein the cylindrical rings are coupled together at a weld point connecting a peak of one cylindrical ring to a valley of an adjacent cylindrical, or wherein each of the cylindrical rings of the trunk portion each comprises a series of peaks and valleys coupled together by substantially straight segments (118), further comprising a link between each of the cylindrical rings coupling adjacent cylindrical rings together.

4. The bifurcated stent of claim 1, wherein the second branch includes a second longitudinally extendable portion, wherein the second longitudinally extendable portion is structured such that a user may increase a length of the second branch prior to installation in a patient.

5. The bifurcated stent of claim 4, wherein the second longitudinally extendable portion includes at least two cylindrical rings and at least one longitudinal link coupling the at least two cylindrical rings together, wherein the at least one longitudinal link includes a curved portion structured such that pulling the second branch causes the longitudinal link to straighten and a distance between the two cylindrical rings to increase, thereby increasing the length of the second branch.

6. The bifurcated stent of claim 1, wherein the plurality of cylindrical rings of the first branch includes a plurality of cylindrical rings associated with the longitudinally extendable portion and a plurality of cylindrical rings not associated with the longitudinally extendable portion,
wherein the plurality of cylindrical rings associated with the longitudinally extendable portion include a series of peaks and valley connected by segments and the plurality of cylindrical rings not associated with the longitudinally extendable portions include a series of peaks and valleys connected by segments, and
wherein the segments of the cylindrical rings of the longitudinally extendable portion are a first length and the segments of the cylindrical rings not associated with the longitudinally extendable portion are a second length, wherein the first length is smaller than the second length, or
wherein each of cylindrical rings of the longitudinally extendable portion include a first quantity of peaks and valleys and each of the cylindrical rings not associated with the longitudinally extendable portion include a second quantity of peaks and valleys, wherein the first quantity is greater than the second quantity.

7. The bifurcated stent of claim 6, wherein the peaks and valleys of the cylindrical rings of the longitudinally extendable portion have a first unexpanded radius of curvature and the peaks and valleys of the cylindrical rings not associated with the longitudinally extendable portion include a second unexpanded radius of curvature, wherein the first radius of curvature is smaller than the second radius of curvature.

8. The bifurcated stent of claim 1, wherein first branch is coupled to the trunk portion at a weld point (124), or further comprising a link coupling the trunk portion to the first branch.

9. A bifurcated stent comprising:
a trunk portion (402), wherein the trunk portion includes a continuous wire (446) forming a cylindrical body, wherein the continuous wire is formed into a zig-zag pattern around a circumference of the cylindrical body;
a first branch (404) coupled to the trunk portion, wherein the first branch includes a second continuous wire (448) forming a first branch cylindrical body, wherein the second continuous wire is formed into a zig-zag pattern around a circumference of the first branch cylindrical body; and
a second branch (406) coupled to the trunk portion, the second branch includes a third continuous wire (450) forming a second branch cylindrical body, wherein the third continuous wire is formed into a zig-zag pattern around a circumference of the second branch cylindrical body; **characterized in that**
the first branch includes a longitudinally extendable portion (408),
wherein the longitudinally extendable portion is structured such that a user may increase a length of the first branch prior to installation in a patient.

10. The bifurcated stent of claim 9, wherein the longitudinally extendable portion is formed from a first portion of the second continuous wire wound spirally at a first pitch, wherein a remainder of the first branch is formed from a second portion of the second continuous wire wound spirally at a second pitch, wherein the first pitch is smaller than the second pitch.

11. The bifurcated stent of claim 10, wherein the longitudinally extendable portion is structured such that the user may pull the first branch to increase the first pitch, thereby lengthening the first branch.

12. The bifurcated stent of claim 9, wherein the second branch includes a second longitudinally extendable portion, wherein the second longitudinally extendable portion is structured such that a user may increase a length of the second branch prior to installation in a patient.

13. The bifurcated stent of claim 12, wherein the second longitudinally extendable portion is formed from a first portion of the third continuous wire wound spirally at a third pitch, wherein a remainder of the second branch is formed from a second portion of the third continuous wire wound spirally at a fourth pitch, wherein the third pitch is smaller than the fourth pitch.

14. The bifurcated stent of claim 13, wherein the second longitudinally extendable portion is structured such that the user may pull the second branch to increase the third pitch, thereby lengthening the second branch.

15. The bifurcated stent of claim 9, wherein first branch is coupled to the trunk portion at a weld point, or further comprising a link coupling the trunk portion to the first branch.

## Patentansprüche

1. Verzweigter Stent, umfassend:
einen Stammabschnitt (102), der eine Vielzahl von zylindrischen Ringen (112a-112d) umfasst, die miteinander gekoppelt sind;
eine erste Verzweigung (104), die mit dem Stammabschnitt gekoppelt ist, wobei die erste Verzweigung eine Vielzahl von zylindrischen Ringen (116a-116e) umfasst, die miteinander gekoppelt sind; und
eine zweite Verzweigung (106), die mit dem Stammabschnitt gekoppelt ist, wobei die zweite Verzweigung eine Vielzahl von zylindrischen Ringen (114a-114e) umfasst, die miteinander gekoppelt sind; **dadurch gekennzeichnet, dass**
die erste Verzweigung einen in Längsrichtung ausdehnbaren Abschnitt (108) umfasst, wobei der in Längsrichtung ausdehnbare Abschnitt derart aufgebaut ist, dass ein Benutzer eine Länge der ersten Verzweigung vor dem Einsetzen bei einem Patienten vergrößern kann.

2. Verzweigter Stent nach Anspruch 1, wobei der in Längsrichtung ausdehnbare Abschnitt mindestens zwei zylindrische Ringe (116a-116c) und mindestens ein Längsverbindungsglied (130), das die mindestens zwei zylindrischen Ringe zusammen koppelt, umfasst, wobei das mindestens eine Längsverbindungsglied einen gekrümmten Abschnitt (132) umfasst, der derart aufgebaut ist, dass das Ziehen an der ersten Verzweigung dazu führt, dass sich das Längsverbindungsglied gerade richtet und sich ein Abstand zwischen den beiden zylindrischen Ringen vergrößert, wodurch die Länge der ersten Verzweigung vergrößert wird.

3. Verzweigter Stent nach Anspruch 1, wobei jeder der zylindrischen Ringe des Stammabschnitts eine Reihe von Spitzen (122) und Tälern (120) umfasst, die durch Segmente (118) zusammen gekoppelt sind, wobei die zylindrischen Ringe an einer Schweißstelle zusammen gekoppelt sind, die eine Spitze eines zylindrischen Ringes mit einem Tal eines angrenzenden zylindrischen Ringes verbindet, oder wobei jeder der zylindrischen Ringe des Stammabschnitts jeweils eine Reihe von Spitzen und Tälern umfasst, die durch im Wesentlichen gerade Segmente (118) zusammen gekoppelt sind, ferner umfassend ein Verbindungsglied zwischen jedem der zylindrischen Ringe, das angrenzende zylindrische Ringe zusammen koppelt.

4. Verzweigter Stent nach Anspruch 1, wobei die zweite Verzweigung einen zweiten in Längsrichtung ausdehnbaren Abschnitt umfasst, wobei der zweite in Längsrichtung ausdehnbare Abschnitt derart aufgebaut ist, dass ein Benutzer eine Länge der zweiten Verzweigung vor dem Einsetzen bei einem Patienten vergrößern kann.

5. Verzweigter Stent nach Anspruch 4, wobei der zweite in Längsrichtung ausdehnbare Abschnitt mindestens zwei zylindrische Ringe und mindestens ein Längsverbindungsglied, das die mindestens zwei zylindrischen Ringe zusammen koppelt, umfasst, wobei das mindestens eine Längsverbindungsglied einen gekrümmten Abschnitt umfasst, der derart aufgebaut ist, dass das Ziehen an der zweiten Verzweigung dazu führt, dass sich das Längsverbindungsglied gerade richtet und sich ein Abstand zwischen den beiden zylindrischen Ringen vergrößert, wodurch die Länge der zweiten Verzweigung vergrößert wird.

6. Verzweigter Stent nach Anspruch 1, wobei die Vielzahl von zylindrischen Ringen der ersten Verzweigung eine Vielzahl von zylindrischen Ringen, die mit dem in Längsrichtung ausdehnbaren Abschnitt verknüpft ist, und eine Vielzahl von zylindrischen Ringen, die nicht mit dem in Längsrichtung ausdehnbaren Abschnitt verknüpft ist, umfasst,
wobei die Vielzahl von zylindrischen Ringen, die mit dem in Längsrichtung ausdehnbaren Abschnitt verknüpft ist, eine Reihe von Spitzen und Tälern umfasst, die von Segmenten verbunden sind, und die Vielzahl von zylindrischen Ringen, die nicht mit dem in Längsrichtung ausdehnbaren Abschnitt verknüpft ist, eine Reihe von Spitzen und Tälern umfasst, die von Segmenten verbunden sind, und
wobei die Segmente der zylindrischen Ringe des in Längsrichtung ausdehnbaren Abschnitts eine erste Länge aufweisen und die Segmente der zylindrischen Ringe, die nicht mit dem in Längsrichtung ausdehnbaren Abschnitt verknüpft sind, eine zweite Länge aufweisen, wobei die erste Länge kürzer ist als die zweite Länge, oder
wobei jeder der zylindrischen Ringe des in Längsrichtung ausdehnbaren Abschnitts eine erste Menge von Spitzen und Tälern umfasst und jeder der zylindrischen Ringe, die nicht mit dem in Längsrichtung ausdehnbaren Abschnitt verknüpft sind, eine zweite Menge von Spitzen und Tälern umfasst, wobei die erste Menge größer ist als die zweite Menge.

7. Verzweigter Stent nach Anspruch 6, wobei die Spitzen und Täler der zylindrischen Ringe des in Längsrichtung ausdehnbaren Abschnitts einen ersten nicht ausgedehnten Krümmungsradius aufweisen und die Spitzen und Täler der zylindrischen Ringe, die nicht mit dem in Längsrichtung ausdehnbaren Abschnitt verknüpft sind, einen zweiten nicht ausgedehnten Krümmungsradius umfassen, wobei der erste Krümmungsradius kleiner ist als der zweite Krümmungsradius.

8. Verzweigter Stent nach Anspruch 1, wobei die erste Verzweigung mit dem Stammabschiitt an einem Schweißpunkt (124) gekoppelt ist, oder ferner ein Verbindungsglied umfasst, das den Stammabschnitt mit der ersten Verzweigung koppelt.

9. Verzweigter Stent, umfassend:
einen Stammabschnitt (402), wobei der Stammabschnitt einen durchgehenden Draht (446) umfasst, der einen zylindrischen Körper bildet, wobei der durchgehende Draht zu einem Zickzackmuster um einen Umfang des zylindrischen Körpers herum gebildet ist;
eine erste Verzweigung (404), die mit dem Stammabschnitt gekoppelt ist, wobei die erste Verzweigung einen zweiten durchgehenden Draht (448) umfasst, der einen ersten zylindrischen Verzweigungskörper bildet, wobei der zweite durchgehende Draht zu einem Zickzackmuster um einen Umfang des ersten zylindrischen Verzweigungskörpers herum gebildet ist; und
eine zweite Verzweigung (406), die mit dem Stammabschnitt gekoppelt ist, wobei die zweite Verzweigung einen dritten durchgehenden Draht (450) umfasst, der einen zweiten zylindrischen Verzweigungskörper bildet, wobei der dritte durchgehende Draht zu einem Zickzackmuster um einen Umfang des zweiten zylindrischen Verzweigungskörpers herum gebildet ist; **dadurch gekennzeichnet, dass**
die erste Verzweigung einen in Längsrichtung ausdehnbaren Abschnitt (408) umfasst, wobei der in Längsrichtung ausdehnbare Abschnitt derart aufgebaut ist, dass ein Benutzer eine Länge der ersten Verzweigung vor dem Einsetzen bei einem Patienten vergrößern kann.

10. Verzweigter Stent nach Anspruch 9, wobei der in Längsrichtung ausdehnbare Abschnitt aus einem ersten Abschnitt des zweiten durchgehenden Drahts gebildet wird, der spiralförmig mit einer ersten Steigung gewickelt wird, wobei ein Rest der ersten Verzweigung aus einem zweiten Abschnitt des zweiten durchgehenden Drahts spiralförmig mit einer zweiten Steigung gewickelt wird, wobei die erste Steigung kleiner ist als die zweite Steigung.

11. Verzweigter Stent nach Anspruch 10, wobei der in Längsrichtung ausdehnbare Abschnitt derart aufgebaut ist, dass der Benutzer an der ersten Verzweigung ziehen kann, um die erste Steigung zu vergrößern, wodurch er die erste Verzweigung verlängert.

12. Verzweigter Stent nach Anspruch 9, wobei die zweite Verzweigung einen zweiten in Längsrichtung ausdehnbaren Abschnitt umfasst, wobei der zweite in Längsrichtung ausdehnbare Abschnitt derart aufgebaut ist, dass ein Benutzer eine Länge der zweiten Verzweigung vor dem Einsetzen bei einem Patienten vergrößern kann.

13. Verzweigter Stent nach Anspruch 12, wobei der zweite in Längsrichtung ausdehnbare Abschnitt aus einem ersten Abschnitt des dritten durchgehenden Drahts gebildet wird, der spiralförmig mit einer dritten Steigung gewickelt wird, wobei ein Rest der zweiten Verzweigung aus einem zweiten Abschnitt des dritten durchgehenden Drahts spiralförmig mit einer vierten Steigung gewickelt wird, wobei die dritte Steigung kleiner ist als die vierte Steigung.

14. Verzweigter Stent nach Anspruch 13, wobei der zweite in Längsrichtung ausdehnbare Abschnitt derart aufgebaut ist, dass der Benutzer an der zweiten Verzweigung ziehen kann, um die dritte Steigung zu vergrößern, wodurch er die zweite Verzweigung verlängert.

15. Verzweigter Stent nach Anspruch 9, wobei die erste Verzweigung mit dem Stammabschnitt an einem Schweißpunkt gekoppelt ist, oder ferner ein Verbindungsglied umfasst, das den Stammabschnitt mit der ersten Verzweigung koppelt.

## Revendications

1. Endoprothèse bifurquée comprenant :
une partie de tronc (102) comprenant une pluralité d'anneaux (112a-112d) cylindriques reliés les uns aux autres ;
une première branche (104) reliée à la partie de tronc, dans laquelle la première branche comprend une pluralité d'anneaux (116a-116e) cylindriques reliés les uns aux autres ; et
une deuxième branche (106) reliée à la partie de tronc, dans laquelle la deuxième branche comprend une pluralité d'anneaux (114a-114e) cylindriques reliés les uns aux autres ; **caractérisée en ce que**
la première branche comprend une partie extensible (108) longitudinalement, dans laquelle la partie extensible longitudinalement est structurée de sorte qu'un utilisateur puisse augmenter une longueur de la première branche avant installation chez un patient.

2. Endoprothèse bifurquée selon la revendication 1, dans laquelle la partie extensible longitudinalement comprend au moins deux anneaux (116a-116c) cylindriques et au moins une liaison (130) longitudinale reliant entre eux les aux moins deux anneaux cylindriques, dans laquelle l'au moins une liaison longitudinale comprend une partie incurvée (132) structurée de telle sorte que le tirage de la première branche entraîne un redressement de la liaison longitudinale et une augmentation de la distance entre les deux anneaux cylindriques, augmentant ainsi la longueur de la première branche.

3. Endoprothèse bifurquée selon la revendication 1, dans laquelle chacun des anneaux cylindriques de la partie de tronc comprend une série de pics (122) et de creux (120) reliés entre eux par des segments (118), dans laquelle les anneaux cylindriques sont reliés entre eux à un point de soudure reliant un pic d'un anneau cylindrique à un creux d'un anneau cylindrique adjacent, ou dans laquelle chacun des anneaux cylindriques de la partie de tronc comprend une série de pics et de creux reliés entre eux par des segments (118) sensiblement droits, comprenant en outre une liaison entre chacun des anneaux cylindriques reliant entre eux des anneaux cylindriques adjacents.

4. Endoprothèse bifurquée selon la revendication 1, dans laquelle la deuxième branche comprend une deuxième partie extensible longitudinalement, dans laquelle la deuxième partie extensible longitudinalement est structurée de sorte qu'un utilisateur puisse augmenter une longueur de la deuxième branche avant installation chez un patient.

5. Endoprothèse bifurquée selon la revendication 4, dans laquelle la deuxième partie extensible longitudinalement comprend au moins deux anneaux cylindriques et au moins une liaison longitudinale reliant entre eux les aux moins deux anneaux cylindriques, dans laquelle l'au moins une liaison longitudinale comprend une partie incurvée structurée de telle sorte que le tirage de la deuxième branche entraîne un redressement de la liaison longitudinale et une augmentation de la distance entre les deux anneaux cylindriques, augmentant ainsi la longueur de la deuxième branche.

6. Endoprothèse bifurquée selon la revendication 1, dans laquelle la pluralité d'anneaux cylindriques de la première branche comprend une pluralité d'anneaux cylindriques associés à la partie extensible longitudinalement et une pluralité d'anneaux cylindriques non associés à la partie extensible longitudinalement,
dans laquelle la pluralité d'anneaux cylindriques associés à la partie extensible longitudinalement comprennent une série de pics et de creux reliés par des segments et la pluralité d'anneaux cylindriques non associés aux parties extensibles longitudinalement comprennent une série de pics et de creux reliés par des segments, et
dans laquelle les segments des anneaux cylindriques de la partie extensible longitudinalement présentent une première longueur et les segments des anneaux cylindriques non associés à la partie extensible longitudinalement présentent une deuxième longueur, dans laquelle la première longueur est inférieure à la deuxième longueur, ou
dans laquelle chacun des anneaux cylindriques de la partie extensible longitudinalement comprend une première quantité de pics et de creux et chacun des anneaux cylindriques non associés à la partie extensible longitudinalement comprend une deuxième quantité de pics et de creux, dans laquelle la première quantité est supérieure à la deuxième quantité.

7. Endoprothèse bifurquée selon la revendication 6, dans laquelle les pics et les creux des anneaux cylindriques de la partie extensible longitudinalement présentent un premier rayon de courbure non étendu et les pics et les creux des anneaux cylindriques non associés à la partie extensible longitudinalement comprennent un deuxième rayon de courbure non étendu, dans laquelle le premier rayon de courbure est inférieur au deuxième rayon de courbure.

8. Endoprothèse bifurquée selon la revendication 1, dans laquelle la première branche est reliée à la partie de tronc à un point de soudure (124), ou comprenant en outre une liaison reliant la partie de tronc à la première branche.

9. Endoprothèse bifurquée comprenant :
une partie de tronc (402), dans laquelle la partie de tronc comprend un fil (446) continu formant un corps cylindrique, dans laquelle le fil continu présente une forme en zigzag autour d'une circonférence du corps cylindrique ;
une première branche (404) reliée à la partie de tronc, dans laquelle la première branche comprend un deuxième fil (448) continu formant un corps cylindrique de première branche, dans laquelle le deuxième fil continu présente une forme en zigzag autour d'une circonférence du corps cylindrique de première branche ; et
une deuxième branche (406) reliée à la partie de tronc, la deuxième branche comprend un troisième fil (450) continu formant un corps cylindrique de deuxième branche, dans laquelle le troisième fil continu présente une forme en zigzag autour d'une circonférence du corps cylindrique de deuxième branche ; **caractérisée en ce que**
la première branche comprend une partie extensible (408) longitudinalement, dans laquelle la partie extensible longitudinalement est structurée de sorte qu'un utilisateur puisse augmenter une longueur de la première branche avant installation chez un patient.

10. Endoprothèse bifurquée selon la revendication 9, dans laquelle la partie extensible longitudinalement est formée à partir d'une première partie du deuxième fil continu enroulé en spirale au niveau d'un premier pas, dans laquelle une partie restante de la première branche est formée à partir d'une deuxième partie du deuxième fil continu enroulé en spirale au niveau d'un deuxième pas, dans laquelle le premier pas est inférieur au deuxième pas.

11. Endoprothèse bifurquée selon la revendication 10, dans laquelle la partie extensible longitudinalement est structurée de telle sorte que l'utilisateur puisse tirer la première branche pour augmenter le premier pas, allongeant ainsi la première branche.

12. Endoprothèse bifurquée selon la revendication 9, dans laquelle la deuxième branche comprend une deuxième partie extensible longitudinalement, dans laquelle la deuxième partie extensible longitudinalement est structurée de sorte qu'un utilisateur puisse augmenter une longueur de la deuxième branche avant installation chez un patient.

13. Endoprothèse bifurquée selon la revendication 12, dans laquelle la deuxième partie extensible longitudinalement est formée à partir d'une première partie du troisième fil continu enroulé en spirale au niveau d'un troisième pas, dans laquelle une partie restante de la deuxième branche est formée à partir d'une deuxième partie du troisième fil continu enroulé en spirale au niveau d'un quatrième pas, dans laquelle le troisième pas est inférieur au quatrième pas.

14. Endoprothèse bifurquée selon la revendication 13, dans laquelle la deuxième partie extensible longitudinalement est structurée de telle sorte que l'utilisateur puisse tirer la deuxième branche pour augmenter le troisième pas, allongeant ainsi la deuxième branche.

15. Endoprothèse bifurquée selon la revendication 9, dans laquelle la première branche est reliée à la partie de tronc à un point de soudure, ou comprenant en outre une liaison reliant la partie de tronc à la première branche.
